# FASCICULE DE BREVET EUROPEEN

(11) **EP 3 302 235 B1**
(45) Date de publication et mention de la délivrance du brevet: **29.01.2025**
(21) Numéro de dépôt: 16729808.2
(22) Date de dépôt: 30.05.2016
(51) Int. Cl.: A61B 5/00, A61B 5/087, A61M 16/00

(54) **DISPOSITIF DE DIAGNOSTIC DE L'EFFICACITE DE LA VENTILATION D'UN PATIENT ET PROCEDE POUR DETERMINER L'EFFICACITE VENTILATOIRE D'UN PATIENT**
VORRICHTUNG ZUR DIAGNOSE DER WIRKSAMKEIT DER BEATMUNG EINES PATIENTEN UND VERFAHREN ZUR BESTIMMUNG DER WIRKSAMKEIT DER ATEMEFFIZIENZ EINES PATIENTEN
DEVICE FOR DIAGNOSING THE EFFICACY OF VENTILATION OF A PATIENT AND METHOD FOR DETERMINING THE VENTILATORY EFFICACY OF A PATIENT

(30) Priorité: 08.06.2015 FR 1555220
(43) Date de publication de la demande: 11.04.2018
(73) Titulaire: ARCHEON, 25000 Besançon (FR); Université de Franche-Comté, 25000 Besançon (FR); Centre Hospitalier Régional Universitaire de Besançon, 25000 Besançon (FR)
(72) Inventeur: KHOURY, Abdo, 25000 Besançon (FR); DE LUCA, Alban, 25000 Besançon (FR); SALL, Fatimata, Seydou, 25000 Besançon (FR); PAZART, Lionel, 25000 Besançon (FR); CAPELLIER, Gilles, 25320 Grandfontaine (FR); SAILLARD, Pierre-Edouard, 25000 Besançon (FR); NITA, Florin, Dan, 25770 Serre-les-Sapins (FR); VINCHANT, Jean-François, 25000 Besançon (FR)
(74) Mandataire: Radzimski, Eric
(86) Numéro de dépôt international: PCT/EP2016/062162
(87) Numéro de publication internationale: WO 2016/198275

(56) Documents cités:
- WO-A1-2014/078840
- FR-A1- 2 988 004
- US-A1- 2010 170 512
- US-A1- 2014 275 820

## Description

La présente invention concerne un dispositif de diagnostic de l'efficacité ventilatoire d'un patient sous assistance respiratoire. L'invention concerne également un système de ventilation pour l'assistance respiratoire d'un patient comportant un tel dispositif ainsi qu'un procédé pour déterminer l'efficacité ventilatoire d'un patient à l'aide d'un tel système de ventilation.

Les systèmes de ventilation sont utilisés par les secouristes et personnels médicaux et paramédicaux intervenant dans les urgences, en anesthésie et réanimation à l'intérieur ou à l'extérieur de l'hôpital ou autre centre de soins.

Plusieurs projets de recherche ont été entrepris et plusieurs dispositifs ont été développés ces dernières années pour améliorer l'efficacité de la ventilation manuelle.

US2013/0180527 traite de l'optimisation de la forme du ballon, utilisé pour la ventilation, comportant des empreintes de doigts afin d'imposer une méthode de compression unique, réduisant ainsi la variation du volume d'air délivré au patient. Cet appareil a été conçu pour fournir un volume constant entre 500 et 600 ml.

US2008/0236585 mesure des débits d'air et la pression de pointe au niveau de la valve d'insufflation, indique aux secouristes les fréquences de ventilation idéales par le biais d'un signal de cadencement lumineux et affiche le volume insufflé à chaque cycle ventilatoire.

US 2010/170512 et US 2014/275820 concernent des dispositifs de réanimation.

WO2014/078840 décrit un système et un procédé pour contrôler la réanimation et la fonction respiratoire d'un patient. Un capteur de pression détecte la pression de l'air et génère un premier signal de détection. Un capteur de débit mesure un débit d'air et génère un deuxième signal. Un processeur reçoit et traite les premier et second signaux de détection en utilisant un algorithme pour identifier une fréquence ventilatoire, une pression pulmonaire et un volume d'air délivré au patient. Un rapport d'analyse est généré en temps réel avec ces valeurs identifiées.

Parmi les dispositifs commercialisés, le Medumat Easy CPR de la société Weinmann est une alternative moins coûteuse aux ventilateurs transportables mécaniques. Ce dispositif fournit une ventilation artificielle à pression positive déclenchée manuellement et dispose d'une fonction de cadencement permettant au secouriste de respecter la fréquence de ventilation optimale telle que décrite dans les recommandations internationales de médecine d'urgence. Ce dispositif a été évalué dans quelques études et diminue *a priori* la dispersion des paramètres ventilatoires tels que la fréquence de ventilation et volumes insufflés. Cependant, son utilisation nécessite une source d'oxygène sous pression. De plus, des compétences du secouriste en physiologie respiratoire et management des voies respiratoires sont nécessaires pour pouvoir ajuster les paramètres ventilatoires en fonction de l'état clinique du patient.

Un autre dispositif connu commercialisé sous la marque Exhalometer^{™} de la société Galemed Corporation est destiné à mesurer le volume courant, la ventilation minute et la fréquence de ventilation délivrée au patient. Ce dispositif mesure la quantité d'air passant à travers la valve d'expiration du ballon, qui peut différer significativement du volume courant réel. En effet, de nombreuses études ont montré une grande quantité de fuites, soit entre 25 et 40% de fuites, pendant la ventilation au masque, de sorte que le volume expiré traversant l'Exhalometer^{™} est tronqué par les fuites qui se produisent au cours de l'insufflation et de l'expiration entre le masque et le visage du patient.

Ces deux dispositifs ne permettent pas d'évaluer l'efficacité de la ventilation prenant en compte l'état clinique du patient et ne disposent pas de fonction pour réduire l'hyperventilation ou pour délivrer des messages d'alerte aux secouristes.

Il existe ainsi un besoin pour disposer d'un système de ventilation qui prenne en compte l'état clinique du patient.

Il existe encore un besoin pour disposer d'un système de ventilation qui soit utilisable par tout secouriste et personnel médical ou paramédical intervenant dans un centre de soins ou à l'extérieur, sans formation approfondie préalable.

Il existe enfin un besoin pour disposer d'un système de ventilation qui permette une correction rapide d'une mauvaise ventilation.

Pour répondre à tout ou partie des besoins précités, la présente invention propose un dispositif de diagnostic de l'efficacité ventilatoire d'un patient placé sous assistance respiratoire, destiné à coopérer avec un système de ventilation du patient, le dispositif comportant :
- un capteur massique thermique bidirectionnel, apte à mesurer en temps réel des débits d'air à l'insufflation et à l'expiration,
- un boîtier électronique connecté audit capteur, configuré pour recevoir et traiter des données relatives aux débits d'air mesurés par le capteur.

Grâce à la présence du capteur massique thermique bidirectionnel, il est possible de mesurer les débits d'air à l'insufflation et à l'expiration par le biais de la mesure d'un gradient de température que l'on corrèle à la quantité du fluide gazeux le traversant. Ce capteur n'oppose pas de résistance significative au flux d'air, que ce soit à l'inspiration ou à l'expiration, et permet une calibration de la mesure en fonction de la température, de la pression, et de la composition du fluide (air, O2, N2) et n'est pas sensible à la gravité ou à l'orientation du dispositif. Contrairement aux débitmètres à gradient de pression utilisés dans les ventilateurs mécaniques actuels, cette technologie a pour avantage d'être à la fois plus précise sans pour autant opposer de résistance à l'insufflation ou de gêne expiratoire pour le patient.

Le capteur est de préférence à usage unique. En variante, le capteur peut être autoclavable. L'utilisation d'un tel capteur massique thermique bidirectionnel à usage unique ou autoclavable permet d'éviter l'utilisation d'un filtre qui constitue une gêne importante à la ventilation et à la mesure des paramètres ventilatoires de par son encombrement et sa résistance aux flux d'air.

Par « assistance respiratoire » ou « assistance ventilatoire », on entend tout type d'assistance respiratoire, qu'elle soit partielle ou totale, l'assistance respiratoire totale étant encore appelée suppléance respiratoire.

Le dispositif de diagnostic peut être associé à tout dispositif de ventilation d'un patient, pour tout type de nécessité ventilatoire et avec tout type d'interface invasive ou non invasive.

Grâce à l'invention, on dispose d'un dispositif de diagnostic de l'efficacité ventilatoire, compact et léger, disposé au plus proche du patient en amont du masque ou de la sonde pour mesurer les paramètres respiratoires du patient. Sa compacité permet de plus un conditionnement réduit. Son faible poids améliore sa maniabilité et son utilisation.

Le dispositif comporte de préférence une connexion amovible entre le capteur et le boîtier électronique.

La connexion entre le capteur et le boîtier électronique peut se faire facilement, sans outil ni savoir-faire particulier, selon une connexion électro-mécanique.

En variante, la liaison entre le capteur et le boîtier électronique est sans fil.

Le boîtier électronique peut comporter :
- une interface utilisateur comprenant un dispositif d'affichage tel qu'un écran et des moyens de saisie de données,
- un centre de traitement de données,
- un moyen d'alimentation électrique tel qu'au moins une batterie.

Lorsqu'il y a présence de batterie(s), il n'y a pas besoin de branchement électrique sur le secteur, ce qui permet d'utiliser le dispositif de diagnostic dans tout endroit.

Le centre de traitement de données fonctionne par exemple selon des algorithmes programmés d'acquisition, de traitement et d'affichage des données, d'analyse de l'efficacité de la ventilation en temps réel et de gestion d'alarmes, notamment tels que décrits ci-après.

Le boîtier électronique peut se présenter sous forme d'un microprocesseur, connecté par une liaison filaire ou non au capteur massique thermique bidirectionnel. L'interface utilisateur et le centre de traitement de données et tout autre composant du boîtier électronique peuvent être situés au sein d'un même appareil ou être dissociés ou à distance l'un de l'autre ou les uns des autres.

Le dispositif de diagnostic peut encore comporter au moins un autre capteur, choisi parmi les capteurs suivants : un capteur de pression, un capteur de concentration de CO₂ dans l'air. De tels capteurs peuvent permettre de mesurer des caractéristiques pulmonaires et des caractéristiques de l'état clinique du patient, qui pourront ensuite être analysées par le centre de traitement de données du boîtier électronique du dispositif. Lorsqu'ils sont présents, le ou les autres capteurs peuvent être intégrés au dispositif de diagnostic. En variante, ils peuvent être présents dans le système de ventilation avec lequel le dispositif de diagnostic coopère.

Le dispositif de diagnostic permet d'évaluer le volume courant réel permettant de contrôler et de donner des informations relatives à la quantité d'air efficace participant à l'échange gazeux. Il réalise une analyse sur mesure et en temps réel de l'efficacité ventilatoire au regard des caractéristiques physiologiques du patient. Le dispositif fournit au secouriste des messages d'alerte et de consigne pour faire en sorte de maintenir une ventilation adéquate en toute circonstance. Le dispositif de diagnostic prend en compte les caractéristiques physiologiques du patient pour donner une information au secouriste sur la fréquence de ventilation adéquate, notamment par un signal lumineux et/ou sonore, et afficher le volume courant efficace qui doit être délivré au patient.

Le dispositif de diagnostic de l'efficacité ventilatoire du patient sous assistance respiratoire permet l'ajustement en temps réel des paramètres ventilatoires prodigués au patient en adéquation avec ses besoins recommandés ou l'évolution de son état clinique.

Par « caractéristiques physiologiques du patient » ou « paramètres physiologiques du patient », on entend toute grandeur physique qui caractérise les propriétés intrinsèques du patient tant au niveau des caractéristiques mécaniques du système respiratoire, telles que la capacité pulmonaire, la compliance pulmonaire, la résistance pulmonaire, la constante de temps expiratoire, entre autres, que des variables résultant de l'interaction entre la ventilation du patient et les autres systèmes physiologiques, et notamment le système cardiovasculaire, telles que la concentration de CO₂ dans l'air expiré, la saturation artérielle en oxygène, entre autres.

Par « paramètres ventilatoires », on entend les paramètres mesurés correspondant à la mise en oeuvre de l'assistance respiratoire au patient.

L'invention a encore pour objet, selon un autre de ses aspects, en combinaison avec ce qui précède, un système de ventilation pour assistance respiratoire d'un patient, comportant un dispositif de diagnostic de l'efficacité de la ventilation d'un patient tel que défini plus haut, et un dispositif de ventilation choisi dans le groupe constitué par : un ballon souple, un ballon auto-remplisseur et un ventilateur mécanique.

Le système de ventilation est de préférence apte à convenir pour une utilisation choisie dans le groupe constitué par : une ventilation continue d'un patient en détresse respiratoire, une suppléance respiratoire d'un patient en apnée, une assistance d'un patient en ventilation spontanée et une ventilation discontinue d'un patient en arrêt cardiaque.

Le système de ventilation comporte avantageusement une interface de ventilation choisie dans le groupe constitué par : une ventilation invasive sur sonde trachéale ou trachéotomie, une ventilation non invasive sur masque.

L'invention a encore pour objet, selon un autre de ses aspects, indépendamment ou en combinaison avec ce qui précède, un procédé pour déterminer l'efficacité ventilatoire d'un patient à l'aide d'un système de ventilation notamment tel que défini plus haut ou de tout autre système de ventilation adéquat, comprenant au moins un dispositif de ventilation, une interface de ventilation et un ou plusieurs capteurs de débit d'air, de pression et/ou de concentration de CO₂ dans l'air et d'un micro-processeur associé, procédé caractérisé par le fait qu'il comporte les étapes suivantes :
a) permettre la saisie de caractéristiques physiques et/ou physiologiques du patient dans le boîtier électronique, et/ou des caractéristiques relatives à la ventilation, notamment relatives au type de ventilation, au type de dispositif de ventilation et/ou au type d'interface de ventilation,
b) mesurer les paramètres physiologiques du patient à l'aide du ou des capteur(s),
c) analyser les caractéristiques saisies à l'étape a) et les paramètres mesurés à l'étape b),
d) en déduire, en temps réel, des paramètres ventilatoires idéaux pour une ventilation optimale dudit patient, et pour chaque paramètre ventilatoire, un seuil minimal et/ou maximal,
e) mesurer en temps réel les paramètres ventilatoires du patient,
f) comparer les paramètres ventilatoires mesurés auxdits seuils, respectivement,
g) pour chaque paramètre ventilatoire, en cas de valeur d'un paramètre ventilatoire mesuré supérieur à un seuil maximal et/ou inférieur à un seuil minimal correspondants, générer une alarme et/ou une information sur le ou les paramètres à modifier ou correctifs à effectuer pour atteindre une ventilation optimale,
h) répéter les étapes b) à g) pendant toute la durée de l'assistance ventilatoire du patient, notamment à chaque cycle de ventilation.

Grâce au procédé selon l'invention, notamment dans ses étapes c) et d), on peut effectuer un diagnostic des caractéristiques physiologiques du patient placé sous assistance respiratoire et réaliser un ajustement en temps réel des paramètres ventilatoires prodigués au patient en adéquation avec ses besoins recommandés ou l'évolution de son état clinique, et un ajustement des seuils d'alarme en conséquence.

Le procédé consiste selon l'invention à réaliser une interprétation automatisée et continue des courbes respiratoires. Un système de gestion des messages d'alerte permet d'avertir le secouriste en cas de ventilation délétère et de lui indiquer la façon la plus efficace de recouvrer une ventilation adéquate. L'objectif est de détecter le paramètre ayant un impact négatif sur l'efficacité ventilatoire et d'afficher des messages spécifiques au secouriste afin de retrouver un niveau d'efficacité satisfaisant le plus rapidement et le plus simplement possible. Plusieurs problèmes peuvent se produire lorsque la ventilation est insuffisante ou excessive et le rôle de cette fonction clé est donc d'indiquer lequel de ces paramètres peut être corrigé en priorité pour assurer une ventilation efficace.

Les caractéristiques et paramètres physiques et/ou physiologiques du patient comprennent avantageusement au moins deux des caractéristiques ou paramètres suivants : la taille du patient, sa capacité pulmonaire, sa compliance pulmonaire, sa résistance pulmonaire, sa constante de temps expiratoire, sa pression positive en fin d'expiration, sa concentration de CO₂ dans l'air expiré.

Les paramètres ventilatoires comportent par exemple au moins deux parmi les paramètres suivants : le volume insufflé, le volume expiré, le volume courant, le volume de fuites, la fréquence ventilatoire et la pression d'insufflation.

Par ailleurs, grâce à l'invention, les paramètres à modifier ou correctifs pour revenir dans une plage de valeurs acceptable sont déterminés pour le secouriste, lui permettant ainsi d'agir en temps réel pour, le cas échéant, modifier le ou les paramètres concernés dans le sens indiqué. Cela permet d'assurer que les paramètres ventilatoires sont optimaux en garantissant ainsi une assistance ventilatoire performante pour le patient, sans nécessité de connaissances particulières de la part du secouriste.

Les paramètres à modifier ou correctifs à effectuer peuvent être transmis à l'utilisateur, c'est-à-dire au secouriste, par l'intermédiaire d'un dispositif d'affichage tel qu'un écran et/ou d'un indicateur visuel et/ou sonore et/ou tactile.

Le procédé de ventilation du patient selon l'invention permet de prendre en compte l'état clinique du patient en temps réel ainsi que ses caractéristiques physiologiques. Cela permet de ventiler le patient au mieux en fonction de son état clinique, au fur et à mesure de l'assistance respiratoire.

Le dispositif de ventilation, l'interface de ventilation du système de ventilation du patient pour la mise en oeuvre du procédé peuvent être tels que définis plus haut. Le ou les capteurs peuvent être tels que définis plus haut. En variante, à la place du capteur massique thermique bidirectionnel, le système de ventilation peut inclure tout autre type de capteur de débit d'air convenable. Le micro-processeur peut être relié de manière filaire ou non au(x) capteur(s). Le micro-processeur peut être similaire au boîtier électronique tel que défini plus haut, étant agencé pour traiter l'information reçue du ou des capteurs et l'information saisie par l'utilisateur, et pour délivrer des informations à celui-ci selon le procédé.

L'invention pourra être mieux comprise à la lecture de la description détaillée qui va suivre, d'un exemple de mise en oeuvre non limitatif de celle-ci, et à l'examen du dessin annexé, sur lequel :
- la figure 1 est un schéma d'un système de ventilation conforme à l'invention, intégrant un dispositif de diagnostic de l'efficacité de la ventilation d'un patient selon l'invention,
- la figure 2 représente de manière schématique et partielle, en perspective, le système de ventilation de la figure 1,
- la figure 3 représente, de manière schématique et isolément, un exemple d'affichage du dispositif d'affichage du boîtier électronique du dispositif de diagnostic de l'efficacité de la ventilation d'un patient de la figure 1 ou 2,
- la figure 4 représente, de manière schématique, les étapes du procédé de ventilation d'un patient selon l'invention, et
- les figures 5 à 7 détaillent respectivement certaines étapes du procédé de la figure 4.

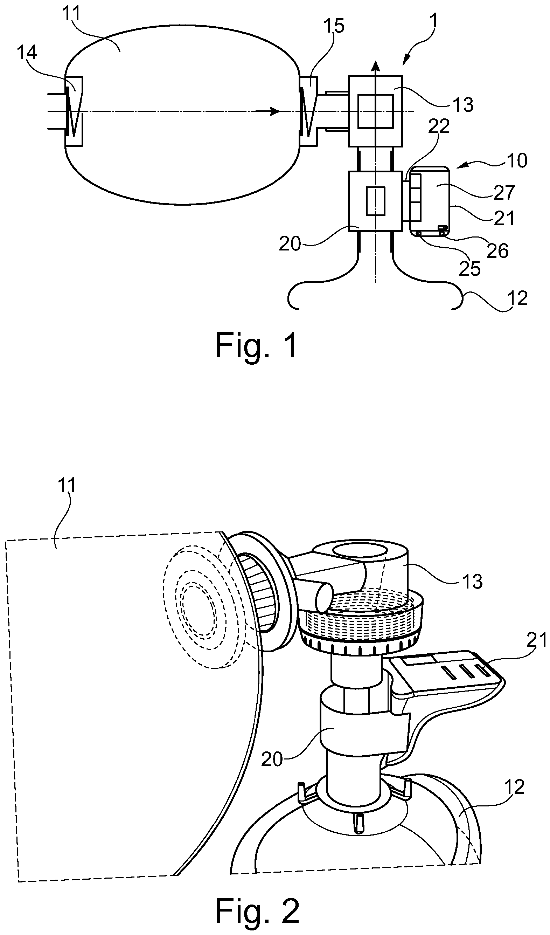

On a représenté sur la figure 1 un système de ventilation 1 pour assistance respiratoire d'un patient 1 comportant un dispositif 10 d'analyse de l'efficacité ventilatoire du patient qui sera décrit ci-après.

Le système de ventilation 1 comporte un dispositif de ventilation 11, formant dans cet exemple un ballon auto-remplisseur. On ne sort pas du cadre de l'invention si le dispositif de ventilation est différent, par exemple consistant en un ventilateur mécanique ou un ballon souple ou autre.

Le système de ventilation 1 peut convenir pour une utilisation telle qu'une ventilation continue d'un patient en détresse respiratoire, une suppléance respiratoire d'un patient en apnée, une assistance d'un patient en ventilation spontanée ou une ventilation discontinue d'un patient en arrêt cardiaque ou autre utilisation.

Le système de ventilation 1 comporte en outre une interface de ventilation 12 servant à relier le système de ventilation 1 au patient, consistant dans l'exemple illustré en une ventilation non invasive sur masque. Le masque est destiné à être appliqué sur la bouche et le nez du patient. On ne sort pas du cadre de l'invention si l'interface de ventilation 12 est constituée par une ventilation invasive sur sonde trachéale ou tout autre dispositif supralaryngé.

Le système de ventilation 1 comporte encore une valve d'expiration unidirectionnelle 13 placée entre le dispositif de ventilation 11 et l'interface de ventilation 12 pour diriger l'air en provenance du dispositif de ventilation 11 vers l'interface de ventilation 12 et laisser échapper l'air expiré par le patient dans l'atmosphère.

Dans cet exemple, le dispositif de ventilation 11 est muni d'une valve anti-retour 14 qui débouche à l'air libre et qui est passante dans le sens de l'atmosphère vers le dispositif de ventilation 11.

Le système de ventilation 1 comporte encore une valve d'insufflation unidirectionnelle 15 qui permet d'alimenter en air le patient.

Le dispositif de diagnostic 10, le dispositif de ventilation 11, l'interface de ventilation 12, la valve d'expiration 13 et la valve d'insufflation 15 sont assemblés entre eux de manière amovible, par exemple par emboîtement comme illustré schématiquement sur la figure 1, de manière connue en soi.

Le dispositif de diagnostic 10 de l'efficacité ventilatoire comporte un capteur 20 massique thermique bidirectionnel apte à mesurer en temps réel des débits d'air à l'insufflation et à l'expiration et un boîtier électronique 21 connecté audit capteur 20 par des moyens de connexion 22 amovibles assurant une connexion électronique et mécanique. Le capteur 20 massique thermique bidirectionnel, encore appelé débitmètre massique thermique, peut être à usage unique ou autoclavable. Il est destiné à être branché comme visible sur les figures 1 et 2, d'un côté, entre la valve d'insufflation 15 du dispositif de ventilation 11 et la valve d'expiration 13, et, de l'autre côté, l'interface de ventilation 12. Le capteur 20 permet de mesurer les débits et les volumes d'air inspiré et expiré en mesurant la capacité calorifique massique du fluide, et par extension la quantité d'air le traversant à chaque cycle de ventilation. Le boîtier électronique 21 est configuré pour recevoir et traiter des données relatives aux débits d'air mesurés par le capteur 20.

Dans l'exemple illustré, le dispositif de diagnostic 10 ne comporte pas d'autre capteur, mais il pourrait en comporter d'autres, par exemple un capteur de pression et/ou un capteur de concentration de CO₂ dans l'air, sans sortir du cadre de l'invention.

Le boîtier électronique 21 du dispositif de diagnostic 10 comporte un centre de traitement de données, incluant une partie informatique ou « hardware » et une partie logicielle ou « software », une interface utilisateur comprenant un dispositif d'affichage et des moyens de saisie de données ou interface de contrôle, et des moyens d'alimentation électrique tels qu'une ou des batteries. Le boîtier électronique 21 permet d'assurer l'interprétation des courbes ventilatoires et d'afficher, pour le secouriste, les informations importantes liées à l'efficacité de la ventilation et différents messages d'alerte. Si l'efficacité de la ventilation est considérée comme inadéquate ou délétère pour le patient, le dispositif de diagnostic 10 permet d'identifier les principales causes de ce manque d'efficacité et envoie des messages d'alerte spécifiques au secouriste.

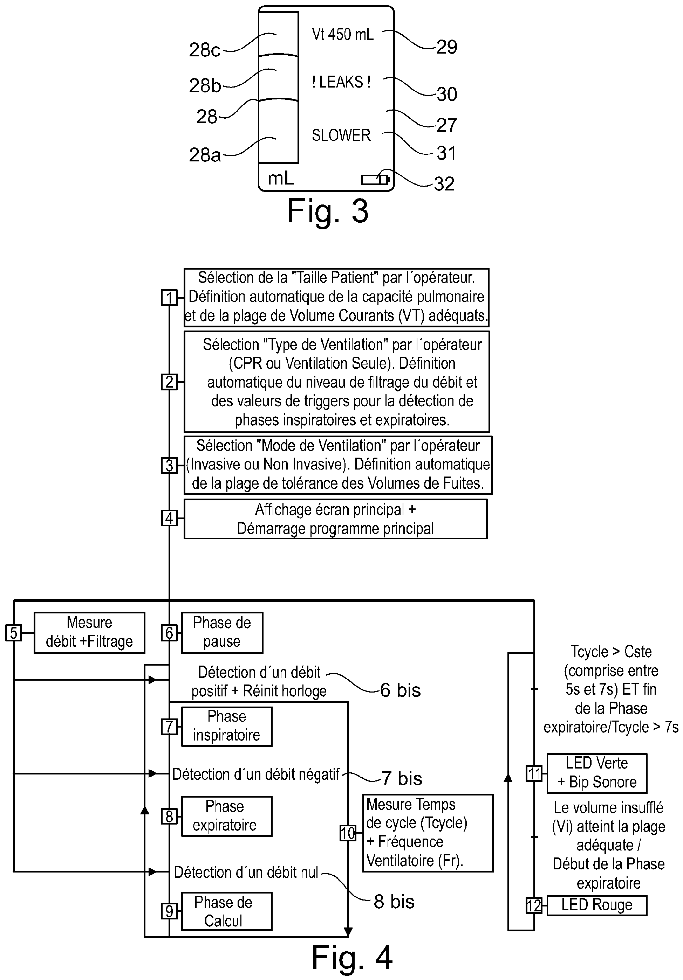

Le boîtier électronique 21 comporte, dans cet exemple, comme visible sur la figure 1, une diode électroluminescente 25 ou LED permettant l'affichage d'une alarme visuelle et un bouton de réinitialisation 26, ainsi qu'un dispositif d'affichage 27 représenté sur la figure 3 permettant l'affichage de différents types d'alertes et messages en fonction de l'analyse de l'efficacité effectuée par le boîtier électronique 21.

Le boîtier électronique 21 peut en variante comporter ou être constitué par une tablette tactile, un ordinateur portable, un téléphone intelligent exécutant une application spécifique, et muni le cas échéant d'une interface matérielle avec le ou les capteurs et autres éléments du système.

L'échange des informations entre le centre de traitement et le ou les capteurs et autres éléments du système peut s'effectuer par voie filaire et/ou non filaire.

Dans l'exemple illustré sur la figure 3, le volume courant Vt 29, qui est le volume d'air atteignant les poumons à chaque respiration, exprimé en ml, est affiché sur le dispositif d'affichage 27 à chaque cycle ventilatoire. Dans cet exemple, on peut lire un volume courant mesuré Vt de 450 ml.

Les volumes inspirés et expirés sont également visualisés sur l'écran sous forme d'un bargraphe 28, divisé en trois parties dans cet exemple, formant trois zones de couleur 28a, 28b et 28c pour indiquer respectivement si le volume est insuffisant (28a), efficace (28b) ou excessif (28c) en fonction des caractéristiques physiologiques du patient.

Les fréquences de ventilation optimales déterminées par le centre de traitement de données, sont transmises au secouriste via un signal lumineux et/ou sonore et/ou tactile pour donner le cadencement adéquat. Dans l'exemple de la figure 3, un message d'alerte 31 indiquant qu'il faut diminuer la fréquence de ventilation apparaît.

Dans l'exemple de la figure 3, un message d'alerte 30 indiquant « leaks » en anglais, c'est-à-dire des fuites, apparaît, informant le secouriste qu'il est nécessaire de réduire les fuites, par exemple en repositionnant le masque du patient. Les fuites sont en effet détectées et calculées en mesurant l'écart entre le volume insufflé et le volume expiré à chaque cycle ventilatoire et/ou constatant une chute de la pression d'insufflation simultanément à une augmentation des débits.

Enfin, toujours sur la figure 3, un indicateur visuel 32 permet de visualiser le niveau de charge de la ou des batteries.

Grâce à ce dispositif de diagnostic 10, on dispose, pour chaque cycle de ventilation, d'un retour d'informations fourni par le boîtier électronique 21 au secouriste sur la valeur des principaux paramètres ventilatoires et sur leur conformité au regard des caractéristiques physiques et physiologiques du patient et des recommandations de l'ILCOR (pour International Liaison Committee On Resuscitation, en anglais, c'est-à-dire le Comité de liaison international sur la réanimation). En effet, la mesure des volumes insufflés et expirés effectuée grâce au capteur 20 placé en amont de l'interface de ventilation 12 permet, après traitement par le centre de traitement de données du boîtier électronique 21, d'estimer, et d'afficher le volume courant, c'est-à-dire la quantité d'air alimentant réellement les poumons du patient, ainsi que les fuites à chaque cycle de ventilation. La mesure des débits permet également la détection des différentes phases du cycle ventilatoire grâce à des déclencheurs (ou « triggers » en anglais) spécifiques. Ces derniers permettent notamment de détecter la fin de la phase d'expiration du patient afin d'éviter l'hyperventilation du patient qui survient lorsque le secouriste réinsuffle le patient avant la fin de l'expiration. Lorsque la détection de la fin de la phase d'expiration n'est pas possible à cause de fuites expiratoires trop importantes, celle-ci peut être estimée grâce à la mesure de la constante de temps expiratoire du patient.

Les figures 4 à 7 illustrent les étapes du procédé de ventilation d'un patient à l'aide du système de ventilation 1, conforme à l'invention.

En référence à la figure 4, le procédé de ventilation d'un patient à l'aide du système de ventilation 1 comporte une étape 1 consistant, pour le secouriste, à utiliser l'interface utilisateur, en particulier les moyens de saisie, pour sélectionner ou indiquer une caractéristique physique et/ou physiologique du patient dans le boîtier électronique 21, en particulier la taille du patient. Le centre de traitement de données, qui récupère la caractéristique, est alors configuré pour définir automatiquement la capacité pulmonaire du patient et la plage de volume courant (Vt) adéquate, c'est-à-dire un seuil minimal et un seuil maximal de volume courant.

Dans une étape 2, le secouriste peut sélectionner ou indiquer une caractéristique relative à la ventilation, notamment le type de ventilation, par exemple au choix parmi la réanimation cardiopulmonaire (encore appelée en anglais CPR pour Cardiopulmonary Resuscitation) ou la ventilation seule. Le centre de traitement de données définit alors automatiquement le niveau de filtrage du débit et des valeurs de déclencheurs pour la détection de phases inspiratoires et expiratoires.

Dans une étape 3, le secouriste peut sélectionner une autre caractéristique de la ventilation, par exemple le mode de ventilation au choix parmi ventilation invasive ou non invasive. Le centre de traitement de données définit alors automatiquement la plage de tolérance des volumes de fuites, c'est-à-dire un seuil maximal de volume de fuites.

Dans une étape 4, l'écran principal du dispositif d'affichage 27 s'allume et le programme principal du centre de traitement de données démarre.

A chaque cycle, une analyse est réalisée.

Dans une étape 5, le débit est mesuré à l'aide du capteur 20 de façon à détecter une phase de pause 6, une phase inspiratoire 7, une phase expiratoire 8 et effectuer une phase de calcul 9. Entre les phases de pause 6 et inspiratoire 7, on a en effet, une étape 6bis, consistant en une détection d'un débit positif générant la réinitialisation de l'horloge, ce qui permet de détecter que l'on est en phase inspiratoire. Par ailleurs, entre les phases inspiratoire 7 et expiratoire 8, on détecte, dans une étape 7bis, un débit négatif, ce qui permet de dire que l'on est en phase expiratoire. Après la phase expiratoire 8, le débit, détecté dans une étape 8bis, est nul, ce qui permet d'enclencher la phase de calcul 9.

A partir de la détection du débit positif jusqu'à la fin du cycle de ventilation, on mesure, dans une étape 10, le temps de cycle (Tcycle) et la fréquence ventilatoire (Fr).

Tout en suivant le cycle de ventilation, et en fonction du résultat obtenu lors de la phase de calcul 9, des informations sont affichées et/ou des alarmes sont enclenchées sous forme d'indicateurs visuels et/ou sonores et/ou tactiles, comme cela sera expliqué plus loin.

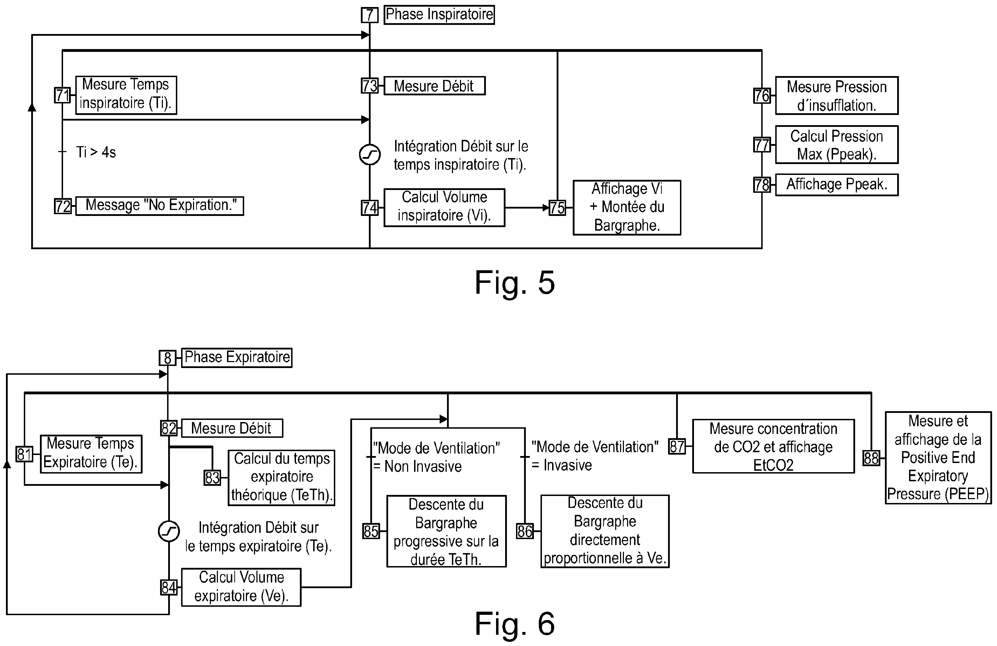

Le détail du procédé lors de la phase inspiratoire 7 est illustré sur la figure 5. Cette phase inspiratoire 7 comprend la mesure du temps inspiratoire Tᵢ 71. Si le temps inspiratoire Tᵢ est supérieur à une durée prédéterminée, par exemple de 4 secondes, un message 72 indiquant « no expiration » en anglais (c'est-à-dire pas d'expiration) est émis. Il est à noter que l'inspiration dure généralement entre 0,5 et 2s. Ainsi, si on ne détecte pas d'expiration après une durée prédéterminée supérieure à 2s, par exemple supérieure à 4s après le début de l'insufflation, on affiche le message 72.

Parallèlement, la mesure du débit, dans une étape 73, est effectuée et on intègre le débit sur le temps respiratoire Tᵢ, ce qui permet, dans une étape 74, de calculer le volume insufflé ou inspiratoire Vᵢ et, dans une étape 75, d'afficher le volume inspiratoire Vᵢ et la montée du bargraphe 28.

Parallèlement, on mesure, dans une étape 76, la pression d'insufflation, on effectue, dans une étape 77, la mesure de la pression maximum Pₚₑₐₖ et, dans une étape 78, l'affichage de cette pression maximum Pₚₑₐₖ est effectué.

Le procédé lors la phase expiratoire 8 est détaillé sur la figure 6. Lors de la phase expiratoire 8, on mesure, dans une étape 81, le temps expiratoire Tₑ.

En parallèle, on mesure le débit, dans une étape 82, et on calcule le temps expiratoire théorique TeTh. Le calcul de TeTh est effectué en évaluant la constante de temps expiratoire du patient qui est égale à 5*R*C, avec R : résistance pulmonaire et C : compliance pulmonaire. TeTh peut également être anticipée par régression exponentielle de la courbe de débit expiratoire. On intègre ensuite le débit sur le temps expiratoire Tₑ pour en déduire le calcul du volume expiratoire Vₑ dans une étape 84. Lorsqu'on est en mode de ventilation non invasive, on effectue dans une étape 85 la descente du bargraphe 28 progressive sur la durée TeTh. Lorsqu'on est en mode de ventilation invasive, on effectue dans une étape 86 la descente du bargraphe 28 directement proportionnelle à Vₑ.

Parallèlement, dans une étape 87, on mesure la concentration de CO₂ et on affiche la quantité de CO₂ expirée EtCO₂, par exemple en utilisant une mesure effectuée par un capteur optionnel placé entre le capteur 20 et l'interface 12. Un tel capteur est par exemple de type NDIR (pour « NonDispersive InfraRed », en anglais, fonctionnant par absorption non dispersive dans l'infrarouge), permettant une mesure par spectroscopie infrarouge.

Parallèlement, dans une étape 88, on mesure et on affiche la pression positive en fin d'expiration, notée PEEP pour « Positive End Expiratory Pressure » en anglais.

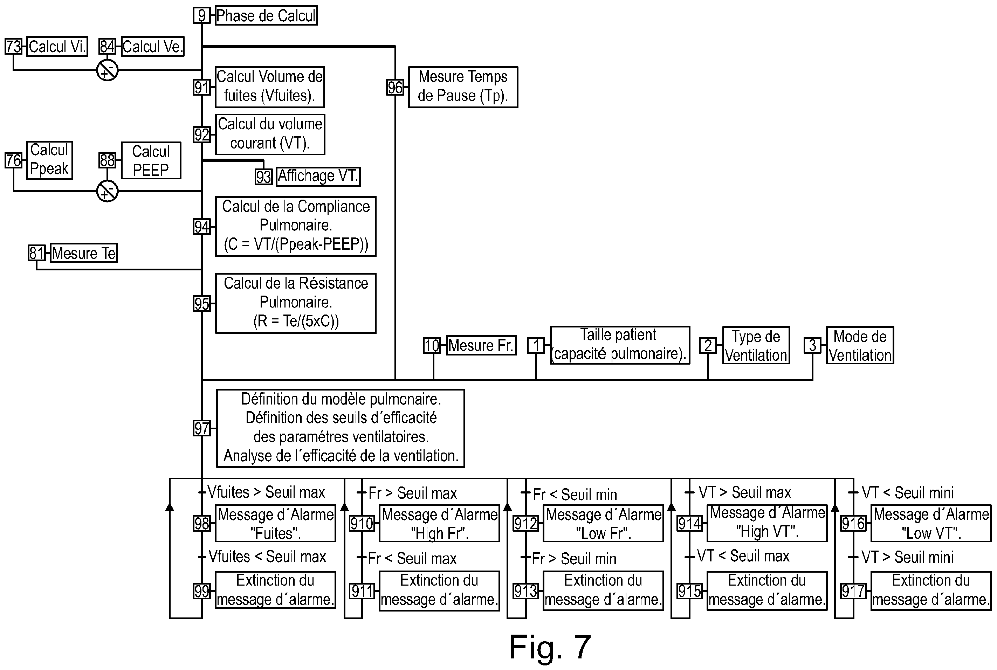

Enfin, au cours de la phase 9 de calcul, comme détaillé sur la figure 7, on calcule, dans une étape 91, le volume de fuites V_{fuites} puis on calcule le volume courant Vt dans une étape 92 et on affiche, dans une étape 93, le volume courant Vt. On calcule dans une étape 94, la compliance pulmonaire C selon la formule C = Vₜ / (Pₚₑₐₖ - PEEP). Dans une étape 95, on calcule la résistance pulmonaire R selon la formule R = Tₑ / 5 * C.

On mesure également, dans une étape 96, le temps de pause Tₚ et, à l'aide de mesure de la fréquence ventilatoire Fr, de la taille du patient, du type de ventilation et du mode de ventilation et des calculs effectués aux étapes 94 et 95 notamment, on définit, dans une étape 97, le modèle pulmonaire ainsi que les seuils d'efficacité et paramètres ventilatoires et on analyse l'efficacité de la ventilation.

Si le volume de fuites V_{fuites} est supérieur à un seuil maximal prédéterminé, alors, dans une étape 98, on affiche un message d'alarme « fuites » ou « leaks » 30. Si le volume de fuites V_{fuites} est inférieur audit seuil maximal prédéterminé, dans une étape 99, il y a extinction du message d'alarme 30.

Parallèlement, si la fréquence ventilatoire Fr est supérieure à un seuil maximal prédéfini, alors, dans une étape 910, on affiche un message d'alarme « fréquence ventilatoire haute » ou « High Fr », mais si la fréquence ventilatoire est inférieure au seuil maximum prédéterminé alors, dans une étape 911, il y a extinction du message d'alarme. Si la fréquence ventilatoire Fr est inférieure à un seuil minimum prédéterminé, alors, dans une étape 912, le message d'alarme « fréquence ventilatoire faible » ou « low Fr » est affiché, mais si la fréquence ventilatoire Fr est supérieure audit seuil minimal prédéterminé, alors, dans une étape 913, il y a extinction du message d'alarme.

Parallèlement, si le volume courant Vt est supérieur à un seuil maximal prédéterminé, alors, dans une étape 914, on affiche le message d'alarme « volume courant élevé » ou « High Vt » mais si le volume courant Vt est inférieur à ce seuil maximal prédéterminé, alors, dans une étape 915, il y a extinction du message d'alarme. Si on a un volume courant Vt inférieur à un seuil minimal prédéterminé, alors, dans une étape 916, est affiché le message d'alarme « volume courant faible » ou « low Vt ». Lorsque le volume courant Vt est supérieur à un seuil minimal prédéterminé alors, dans une étape 917, il y a extinction du message d'alarme.

Dans une étape 11 illustrée sur la figure 4, l'allumage d'une diode électroluminescente 25 de couleur verte et l'émission d'un signal sonore sont effectués lorsque le temps de cycle est supérieur à une constante comprise dans une plage de valeurs prédéterminée, par exemple entre 5 et 7 secondes, et que la fin de la phase expiratoire est détectée, ou que le temps de cycle dépasse une valeur seuil prédéterminée, par exemple 7 secondes. Ce signal sonore et lumineux permet d'indiquer au secouriste l'instant propice à l'insufflation. Lorsque le volume insufflé Vi atteint la plage adéquate ou que le début de la phase expiratoire est détectée, alors, dans une étape 12, l'indicateur visuel tel qu'une diode électroluminescente 25 de couleur rouge est allumé pour avertir le secouriste. La plage adéquate du volume insufflé Vi est déterminée lors des étapes 1 et 97. Le volume Vi est adéquat si les fuites sont nulles. Sinon, le volume adéquat est corrigé en fonction des fuites. Le temps de cycle optimal est basé sur les caractéristiques pulmonaires du patient telles que la compliance et la résistance pulmonaires.

Le volume de fuites peut également être exprimé en pourcentage du volume insufflé et a un seuil maximal prédéterminé, par exemple compris entre 20% et 40% environ du volume insufflé. Le seuil maximal de fréquence respiratoire Fr est par exemple compris entre 12 et 20 cycles environ par minute et le seuil minimal de fréquence ventilatoire Fr est par exemple compris entre 8 et 12 cycles environ par minute. Quant au volume courant Vt, le seuil maximal prédéterminé est par exemple compris entre 500 ml et 700 ml environ et le seuil minimal prédéterminé est par exemple compris entre 300 ml et 500 ml environ.

Grâce à l'invention, le secouriste peut immédiatement avoir une information sur le volume de fuites, la fréquence ventilatoire Fr, le volume courant Vt et influer très rapidement sur le ou les paramètre(s) à corriger, le cas échéant, afin de rétablir une ventilation optimale pour le patient. L'itération des étapes du procédé à chaque cycle de ventilation du patient permet au secouriste de s'adapter en permanence à l'évolution de l'état clinique du patient et de moduler les paramètres indiqués sur le dispositif d'affichage 27, sans avoir de connaissances approfondies du système de ventilation ou de la physiologie respiratoire.

L'invention n'est bien sûr pas limitée à l'exemple qui vient d'être décrit.

En particulier, le système peut être adapté à un usage pédiatrique ou néonatal et les seuils décrits ci-dessus peuvent changer en conséquence.

Dans toute la description, l'expression « comportant un » doit être comprise comme étant synonyme de l'expression « comprenant au moins un ».

Les plages de valeurs sont entendues avec bornes comprises sauf si le contraire est spécifié.

## Revendications

1. Dispositif (10) de diagnostic de l'efficacité de la ventilation d'un patient sous assistance respiratoire, destiné à coopérer avec un système de ventilation (1) du patient comportant un ballon souple ou un ballon auto-remplisseur, le dispositif (10) comportant :
- un capteur (20) massique thermique bidirectionnel, apte à mesurer en temps réel des débits d'air à l'insufflation et à l'expiration,
- un boîtier électronique (21) connecté audit capteur (20), la connexion entre le capteur et le boîtier électronique se faisant selon une connexion électromécanique amovible, le boîtier électronique (21) étant configuré pour recevoir et traiter des données relatives aux débits d'air mesurés par le capteur (20), le boîtier électronique (21) comportant :
i. une interface utilisateur comprenant un dispositif d'affichage (27) et des moyens de saisie de données,
ii. un centre de traitement de données, le centre de traitement de données fonctionnant selon des algorithmes programmés d'acquisition, de traitement et d'affichage des données, d'analyse de l'efficacité de la ventilation en temps réel et de gestion d'alarmes, et
iii. un moyen d'alimentation électrique.

2. Dispositif (10) selon la revendication 1, le dispositif d'affichage (27) étant un écran et le moyen d'alimentation électrique étant une batterie.

3. Dispositif (10) selon l'une quelconque des revendications 1 à 2, le capteur (20) étant à usage unique.

4. Dispositif (10) selon l'une quelconque des revendications 1 à 3, comportant au moins un autre capteur, choisi parmi les capteurs suivants : un capteur de pression, un capteur de concentration de CO₂ dans l'air.

5. Dispositif selon l'une quelconque des revendications précédentes, les moyens de saisie de données étant configurés pour permettre la saisie de caractéristiques physiques et/ou physiologiques du patient dans le boîtier électronique (21), et/ou des caractéristiques relatives à la ventilation, notamment relatives au type de ventilation, au type de dispositif de ventilation (11) et/ou au type d'interface de ventilation (12).

6. Dispositif selon la revendication précédente, les caractéristiques physiques et/ou physiologiques du patient et paramètres physiologiques du patient mesurés à l'aide du ou des capteur(s) comprenant au moins deux parmi les caractéristiques ou paramètres suivants : la taille du patient, sa capacité pulmonaire, sa compliance pulmonaire, sa résistance pulmonaire, sa constante de temps expiratoire, sa pression positive en fin d'expiration, sa concentration de CO2 dans l'air expiré.

7. Dispositif selon la revendication 5 ou 6, le centre de traitement de données étant configuré pour, pendant toute la durée de l'assistance ventilatoire du patient, notamment à chaque cycle de ventilation, analyser lesdites caractéristiques ainsi que les paramètres physiologiques mesurés, notamment à chaque cycle de ventilation, par le capteur (20), afin d'en déduire des paramètres ventilatoires idéaux pour une ventilation optimale dudit patient, et pour chaque paramètre ventilatoire, un seuil minimal et/ou maximal, les paramètres ventilatoires comportant de préférence au moins deux parmi les paramètres suivants : le volume insufflé, le volume expiré, le volume courant (Vt), le volume de fuites, la fréquence ventilatoire (Fr) et la pression d'insufflation.

8. Dispositif selon la revendication 6 ou 7, le centre de traitement de données étant configuré pour recueillir les paramètres ventilatoires mesurés par le capteur (20) et éventuellement par un autre capteur et les comparer auxdits seuils, pendant toute la durée de l'assistance ventilatoire du patient, notamment à chaque cycle de ventilation, , le centre de traitement de données étant de préférence configuré pour, pendant toute la durée de l'assistance ventilatoire du patient, notamment à chaque cycle de ventilation, et pour chaque paramètre ventilatoire, en cas de valeur d'un paramètre ventilatoire mesuré supérieure à un seuil maximal et/ou inférieure à un seuil minimal correspondants, générer une alarme et/ou une information sur le ou les paramètres ventilatoires à modifier ou correctifs à effectuer pour atteindre une ventilation optimale

9. Dispositif selon la revendication précédente, **caractérisé en ce que** le boîtier électronique est configuré pour transmettre à l'utilisateur *via* le dispositif d'affichage (27) les paramètres à modifier ou correctifs et/ou le boîtier électronique comporte un indicateur visuel et/ou sonore et/ou tactile et est configuré pour transmettre à l'utilisateur *via* ledit indicateur les paramètres à modifier ou correctifs.

10. Dispositif selon l'une quelconque des revendications précédentes, dans lequel le centre de traitement de données est configuré pour déduire, en temps réel, des paramètres ventilatoires idéaux pour une ventilation optimale dudit patient, et pour chaque paramètre ventilatoire, un seuil minimal et/ou maximal et, pour chaque paramètre ventilatoire, en cas de valeur d'un paramètre ventilatoire mesuré supérieur à un seuil maximal et/ou inférieur à un seuil minimal correspondants, générer une alarme.

11. Système de ventilation (1) pour assistance respiratoire d'un patient, comportant un dispositif (10) de diagnostic de l'efficacité de la ventilation du patient selon l'une quelconque des revendications 1 à 10, et un dispositif de ventilation (11) choisi dans le groupe constitué par : un ballon souple et un ballon auto-remplisseur, le système de ventilation comportant en outre de préférence une interface de ventilation (12) choisie dans le groupe constitué par : une ventilation invasive sur sonde trachéale ou trachéotomie, une ventilation non invasive sur masque, le capteur (20) massique thermique bidirectionnel étant situé entre le dispositif de ventilation (11) et l'interface de ventilation (12).

12. Procédé pour déterminer l'efficacité de la ventilation d'un patient à l'aide d'un système de ventilation selon la revendication 11, **caractérisé par le fait qu'**il comporte les étapes suivantes :
a) permettre la saisie de caractéristiques physiques et/ou physiologiques du patient dans le boîtier électronique (21), et/ou des caractéristiques relatives à la ventilation, notamment relatives au type de ventilation, au type de dispositif de ventilation (11) et/ou au type d'interface de ventilation (12),
b) mesurer les paramètres physiologiques du patient à l'aide du capteur (20),
c) analyser les caractéristiques saisies à l'étape a) et les paramètres mesurés à l'étape b),
d) en déduire, en temps réel, des paramètres ventilatoires idéaux pour une ventilation optimale dudit patient, et pour chaque paramètre ventilatoire, un seuil minimal et/ou maximal,
e) mesurer en temps réel les paramètres ventilatoires du patient,
f) comparer les paramètres ventilatoires mesurés auxdits seuils, respectivement,
g) pour chaque paramètre ventilatoire, en cas de valeur d'un paramètre ventilatoire mesuré supérieure à un seuil maximal et/ou inférieure à un seuil minimal correspondants, générer une alarme et/ou une information sur le ou les paramètres à modifier ou correctifs à effectuer pour atteindre une ventilation optimale,
h) répéter les étapes b) à g) pendant toute la durée de l'assistance ventilatoire du patient, notamment à chaque cycle de ventilation.

13. Procédé selon la revendication 12, dans lequel les caractéristiques physiques et/ou physiologiques du patient comprennent au moins deux parmi les caractéristiques ou paramètres suivants : la taille du patient, sa capacité pulmonaire, sa compliance pulmonaire, sa résistance pulmonaire, sa constante de temps expiratoire, sa pression positive en fin d'expiration, sa concentration de CO2 dans l'air expiré.

14. Procédé selon la revendication 12 ou 13, dans lequel les paramètres ventilatoires comportent au moins deux parmi les paramètres suivants : le volume insufflé, le volume expiré, le volume courant (Vt), le volume de fuites, la fréquence ventilatoire (Fr) et la pression d'insufflation.

15. Procédé selon l'une quelconque des revendications 12 à 14, dans lequel les paramètres à modifier ou correctifs sont transmis à l'utilisateur par l'intermédiaire d'un dispositif d'affichage (27) tel qu'un écran et/ou d'un indicateur visuel et/ou sonore et/ou tactile.

## Patentansprüche

1. Vorrichtung (10) zur Diagnose der Wirksamkeit der Beatmung eines Patienten unter Atemunterstützung, die dazu bestimmt ist, mit einem Beatmungssystem (1) des Patienten zusammenzuwirken, das einen nachgiebigen Beatmungsbeutel oder einen selbstaufblasenden Beutel umfasst, wobei die Vorrichtung (10) umfasst:
- einen bidirektionalen thermischen Massensensor (20), der in der Lage ist, die Luftströme bei der Insufflation und dem Ausatmen in Echtzeit zu messen,
- eine Elektronikeinheit (21), die mit dem genannten Sensor (20) verbunden ist, wobei die Verbindung zwischen dem Sensor und der Elektronikeinheit als abnehmbare elektromechanische Verbindung vorliegt, wobei die Elektronikeinheit (21) dazu konfiguriert ist, Daten bezüglich der von dem Sensor (20) gemessenen Luftströme zu empfangen und zu verarbeiten, wobei die Elektronikeinheit (21) umfasst:
i. eine Benutzerschnittstelle, die eine Anzeigevorrichtung (27) und Mittel zur Datenerfassung beinhaltet,
ii. ein Datenverarbeitungszentrum, wobei das Datenverarbeitungszentrum gemäß programmierten Algorithmen zur Erfassung, Verarbeitung und Anzeige von Daten, zur Analyse der Wirksamkeit der Beatmung in Echtzeit und zur Verwaltung von Alarmen arbeitet, und
iii. ein Stromversorgungsmittel.

2. Vorrichtung (10) nach Anspruch 1, wobei die Anzeigevorrichtung (27) ein Bildschirm ist und das Stromversorgungsmittel eine Batterie ist.

3. Vorrichtung (10) nach einem beliebigen der Ansprüche 1 bis 2, wobei der Sensor (20) für den einmaligen Gebrauch bestimmt ist.

4. Vorrichtung (10) nach einem beliebigen der Ansprüche 1 bis 3, die mindestens einen anderen Sensor umfasst, der unter den folgenden Sensoren ausgewählt ist: einem Drucksensor, einem Sensor für die Konzentration von CO₂ in der Luft.

5. Vorrichtung nach einem beliebigen der vorstehenden Ansprüche, wobei die Datenerfassungsmittel dazu konfiguriert sind, die Erfassung von physischen und/oder physiologischen Eigenschaften des Patienten in der Elektronikeinheit (21), und/oder von Eigenschaften, die sich auf die Beatmung beziehen, die sich insbesondere auf die Art der Beatmung, die Art der Beatmungsvorrichtung (11) und/oder die Art der Beatmungsschnittstelle (12) beziehen, zu ermöglichen.

6. Vorrichtung nach dem vorstehenden Anspruch, wobei die physischen und/oder physiologischen Eigenschaften des Patienten und physiologischen Parameter des Patienten, die mithilfe des Sensors oder der Sensoren gemessen werden, mindestens zwei unter den folgenden Eigenschaften oder Parameter beinhalten: der Größe des Patienten, seiner Lungenkapazität, seiner Lungencompliance, seinem Lungenwiderstand, seiner exspiratorische Zeitkonstante, seinem positiven endexspiratorischen Druck, seiner Konzentration von CO₂ in der ausgeatmeten Luft.

7. Vorrichtung nach Anspruch 5 oder 6, wobei das Datenverarbeitungszentrum dazu konfiguriert ist, während der gesamten Dauer der Beatmungsunterstützung des Patienten, insbesondere bei jedem Beatmungszyklus, die genannten Eigenschaften sowie die physiologischen Parameter, die insbesondere bei jedem Beatmungszyklus durch den Sensor (20) gemessen werden, zu analysieren, um daraus ideale Beatmungsparameter für eine optimale Beatmung des genannten Patienten und für jeden Beatmungsparameter einen minimalen und/oder maximalen Schwellenwert abzuleiten, wobei die Beatmungsparameter vorzugsweise mindestens zwei unter den folgenden Parameter umfassen: dem insufflierten Volumen, dem ausgeatmeten Volumen, dem Tidalvolumen (Vt), dem Leckagevolumen, der Beatmungsfrequenz (Fr) und dem Insufflationsdruck.

8. Vorrichtung nach Anspruch 6 oder 7, wobei das Datenverarbeitungszentrum dazu konfiguriert ist, die von dem Sensor (20) und möglicherweise von einem weiteren Sensor gemessenen Beatmungsparameter zu sammeln und sie während der gesamten Dauer der Beatmungsunterstützung des Patienten, insbesondere bei jedem Beatmungszyklus, mit den genannten Schwellenwerten zu vergleichen, wobei das Datenverarbeitungszentrum vorzugsweise dazu konfiguriert ist, um während der gesamten Dauer der Beatmungsunterstützung des Patienten, insbesondere bei jedem Beatmungszyklus, und für jeden Beatmungsparameter, im Fall, dass der Wert eines gemessenen Beatmungsparameters über einem entsprechenden maximalen Schwellenwert und/oder unter einem entsprechenden minimalen Schwellenwert liegt, einen Alarm und/oder Informationen über den oder die zu ändernden Beatmungsparameter oder die vorzunehmenden Korrekturen zu erzeugen, um eine optimale Beatmung zu erreichen

9. Vorrichtung nach dem vorstehenden Anspruch, **dadurch gekennzeichnet, dass** die Elektronikeinheit dazu konfiguriert ist, dem Benutzer über die Anzeigevorrichtung (27) die zu ändernden Parameter oder die Korrekturen zu übertragen und/oder die Elektronikeinheit einen visuellen und/oder akustischen und/oder taktilen Indikator umfasst und dazu konfiguriert ist, dem Benutzer über den genannten Indikator die zu ändernden Parameter oder die Korrekturen zu übertragen.

10. Vorrichtung nach einem der vorstehenden Ansprüche, wobei das Datenverarbeitungszentrum dazu konfiguriert ist, in Echtzeit ideale Beatmungsparameter für eine optimale Beatmung des genannten Patienten und für jeden Beatmungsparameter einen minimalen und/oder maximalen Schwellenwert abzuleiten und für jeden Beatmungsparameter im Fall eines Werts eines gemessenen Beatmungsparameters, der größer als ein entsprechender maximaler Schwellenwert und/oder kleiner als ein entsprechender minimaler Schwellenwert ist, einen Alarm zu erzeugen.

11. Beatmungssystem (1) zur Atemunterstützung eines Patienten, umfassend eine Vorrichtung (10) zur Diagnose der Wirksamkeit der Beatmung des Patienten nach einem der Ansprüche 1 bis 10, und eine Beatmungsvorrichtung (11), die ausgewählt ist aus der Gruppe bestehend aus: einem nachgiebigen Beatmungsbeutel und einem selbstaufblasenden Beutel, wobei das Beatmungssystem vorzugsweise weiter eine Beatmungsschnittstelle (12) umfasst, die ausgewählt ist aus der Gruppe bestehend aus: einer invasiven Beatmung über einen Trachealtubus oder ein Tracheostoma, einer nichtinvasiven Beatmung über eine Maske, wobei sich der bidirektionale thermische Massensensor (20) zwischen der Beatmungsvorrichtung (11) und der Beatmungsschnittstelle (12) befindet.

12. Verfahren zur Bestimmung der Wirksamkeit der Beatmung eines Patienten mithilfe eines Beatmungssystems nach Anspruch 11, **dadurch gekennzeichnet, dass** es die folgenden Schritte umfasst:
a) Ermöglichen der Erfassung von physischen und/oder physiologischen Eigenschaften des Patienten in der Elektronikeinheit (21), und/oder von Eigenschaften, die sich auf die Beatmung beziehen, die sich insbesondere auf die Art der Beatmung, die Art der Beatmungsvorrichtung (11) und/oder die Art der Beatmungsschnittstelle (12) beziehen,
b) Messen der physiologischen Parameter des Patienten mithilfe des Sensors (20),
c) Analysieren der in Schritt a) erfassten Eigenschaften und der in Schritt b) gemessenen Parameter,
d) daraus Ableiten idealer Beatmungsparameter für eine optimale Beatmung des genannten Patienten und für jeden Beatmungsparameter eines minimalen und/oder maximalen Schwellenwerts in Echtzeit,
e) Messen der Beatmungsparameter des Patienten in Echtzeit,
f) Vergleichen der gemessenen Beatmungsparameter mit den jeweiligen genannten Schwellenwerten,
g) Erzeugen, für jeden Beatmungsparameter, im Falle eines Wertes eines gemessenen Beatmungsparameters, der höher als ein entsprechender maximaler Schwellenwert und/oder niedriger als ein entsprechender minimaler Schwellenwert ist, eines Alarms und/oder von Informationen über den oder die Parameter, die zu ändern sind oder die Korrekturen, die zu vornehmen sind, um eine optimale Beatmung zu erreichen,
h) Wiederholen der Schritte b) bis g) während der gesamten Dauer der Beatmungsunterstützung des Patienten, insbesondere bei jedem Beatmungszyklus.

13. Verfahren nach Anspruch 12, wobei die physischen und/oder physiologischen Eigenschaften des Patienten mindestens zwei unter den folgenden Eigenschaften oder Parameter beinhalten: der Größe des Patienten, seiner Lungenkapazität, seiner Lungencompliance, seinem Lungenwiderstand, seiner exspiratorische Zeitkonstante, seinem positiven endexspiratorischen Druck, seiner Konzentration von CO₂ in der ausgeatmeten Luft.

14. Verfahren nach Anspruch 12 oder 13, wobei die Beatmungsparameter mindestens zwei unter den folgenden Parametern umfassen: dem insufflierten Volumen, dem ausgeatmeten Volumen, dem Tidalvolumen (Vt), dem Leckagevolumen, der Beatmungsfrequenz (Fr) und dem Insufflationsdruck.

15. Verfahren nach einem der Ansprüche 12 bis 14, wobei die zu ändernden Parameter oder die Korrekturen mittels einer Anzeigevorrichtung (27), wie einem Bildschirm und/oder einem visuellen und/oder akustischen und/oder taktilen Indikator, an den Benutzer übertragen werden.

## Claims

1. Device (10) for assessing the effectiveness of ventilation in a patient under respiratory assistance, designed to interface with a patient ventilation system (1) comprising a flow-inflating bag or a self-inflating bag, the device (10) comprising:
- a bidirectional thermal mass sensor (20) for measuring, in real time, the airflows during insufflation and during expiration,
- an electronic unit (21) connected to said sensor (20), the connection between the sensor and the electronic unit being made by a removable electromechanical connection, the electronic unit (21) being configured to receive and process data relating to the airflows measured by the sensor (20), the electronic unit (21) comprising:
i. a user interface comprising a display device (27) and data input means,
ii. a data-processing centre, the data-processing centre functioning according to programmed algorithms for acquiring, processing and displaying data, for analysing the effectiveness of the ventilation in real time, and for managing alarms, and
iii. a means for supplying electricity.

2. Device (10) according to claim 1, the display device (27) being a screen and the means for supplying electricity being a battery.

3. Device (10) according to any one of claims 1 to 2, the sensor (20) being single-use.

4. Device (10) according to any one of claims 1 to 3, comprising at least one other sensor, chosen from among the following sensors: a pressure sensor, an in-air CO₂ concentration sensor.

5. Device according to any one of the preceding claims, the data input means being configured to enable the input of physical and/or physiological features of the patient in the electronic unit (21), and/or features relating to ventilation, in particular relating to the type of ventilation, to the type of ventilation device (11) and/or to the type of airway device (12).

6. Device according to the preceding claim, the physical and/or physiological features of the patient and physiological parameters of the patient measured using the one or more sensor(s) comprising at least two from among the following characteristics or parameters: height of the patient, lung capacity, lung compliance, lung resistance, expiratory time constant, positive end-expiratory pressure, and CO₂ concentration in the expired air.

7. Device according to claim 5 or 6, the data-processing centre being configured to, for the entire duration of the respiratory assistance of the patient, in particular in each ventilation cycle, analyse said features, as well as the physiological parameters measured, in particular in each ventilation cycle, by the sensor (20), in order to deduce ideal ventilatory parameters from it for an optimal ventilation of said patient, and for each ventilatory parameter, a minimum and/or maximum threshold, the ventilatory parameters preferably comprising at least two from among the following parameters: the insufflated volume, the expired volume, the tidal volume (Vt), the leakage volume, the ventilatory frequency (Fr) and the inspiratory pressure.

8. Device according to claim 6 or 7, the data-processing centre being configured to collect the ventilatory parameters measured by the sensor (20), and optionally by another sensor and compare them with said thresholds, for the entire duration of the respiratory assistance of the patient, in particular in each ventilation cycle, the data-processing centre preferably being configured to, for the entire duration of the respiratory assistance of the patient, in particular upon each ventilation cycle, and for each ventilatory parameter, in case of value of a measured ventilatory parameter greater than a corresponding maximum threshold and/or less than a corresponding minimum threshold, generate an alarm and/or information about the ventilatory parameter(s) to be modified or adjustments to be made to achieve an optimal ventilation.

9. Device according to the preceding claim, **characterised in that** the electronic unit is configured to transmit to the user, via the display device (27), the parameters to be modified or adjustments to be made and/or the electronic unit comprises a visual and/or audio and/or tactile indicator, and is configured to transmit to the user via said indicator, the parameters to be modified or adjustments to be made.

10. Device according to any one of the preceding claims, wherein the data-processing centre is configured to deduce, in real time, ideal ventilatory parameters for an optimal ventilation of said patient, and for each ventilatory parameter, a minimum and/or maximum threshold and, for each ventilatory parameter, in case of value of a measured ventilatory parameter greater than a corresponding maximum threshold and/or less than a corresponding minimum threshold, generate an alarm.

11. Ventilation system (1) for the respiratory assistance of a patient, comprising a device (10) for assessing the effectiveness of ventilation in the patient according to any one of claims 1 to 10, and a ventilation device (11) chosen from the group consisting of: a flow-inflating bag and a self-inflating bag, the ventilation system further preferably comprising an airway device (12) chosen from the group consisting of: invasive ventilation via tracheal tube or tracheotomy, non-invasive ventilation via mask, with the bidirectional thermal mass sensor (20) being located between the ventilation device (11) and the airway device (12).

12. Method for assessing the effectiveness of ventilation in a patient using a ventilation system according to claim 11, **characterised in that** it comprises the following steps:
a) enabling the input of physical and/or physiological features of the patient in the electronic unit (21), and/or features relating to ventilation, in particular relating to the type of ventilation, to the type of ventilation device (11) and/or to the type of airway device (12),
b) measuring the physiological parameters of the patient using the sensor (20),
c) analysing the features entered in step a) and the parameters measured in step b),
d) deducing from this, in real time, ideal ventilatory parameters for an optimal ventilation of said patient, and for each ventilatory parameter, a minimum and/or maximum threshold,
e) measuring, in real time, the ventilatory parameters of the patient,
f) comparing the measured ventilatory parameters with said thresholds, respectively,
g) for each ventilatory parameter, in case of value of a measured ventilatory parameter greater than a corresponding maximum threshold and/or less than a corresponding minimum threshold, generating an alarm and/or information about the parameter(s) to be modified or correction(s) to be made to achieve an optimal ventilation,
h) repeating steps b) to g) for the entire duration of the respiratory assistance of the patient, in particular in each ventilation cycle.

13. Method according to claim 12, wherein the physical and/or physiological features of the patient comprise at least two from among the following features or parameters: height of the patient, lung capacity, lung compliance, lung resistance, expiratory time constant, positive end-expiratory pressure, and CO₂ concentration in the expired air.

14. Method according to claim 12 or 13, wherein the ventilatory parameters comprise at least two from among the following parameters: the insufflated volume, the expired volume, the tidal volume (Vt), the leakage volume, the ventilatory frequency (Fr) and the inspiratory pressure.

15. Method according to any one of claims 12 to 14, wherein the parameters to be modified or corrections are transmitted to the user through a display device (27), such as a screen and/or a visual and/or audio and/or tactile indicator.
